# FASCICULE DE BREVET EUROPEEN

(11) **EP 0 669 118 B1**
(45) Date de publication et mention de la délivrance du brevet: **21.11.2001**
(21) Numéro de dépôt: 95400241.6
(22) Date de dépôt: 06.02.1995
(51) Int. Cl.: A61F 5/453, A61F 6/00

(54) **Applicateur pour un cathéter externe de collecte urinaire et dispositif de collecte urinaire comportant ledit applicateur**
Anlegevorrichtung für einen externen Harnkatheter und Harnkatheter der eine solche Anlegevorrichtung enthält
Applicator for an external urinary catheter and urinary catheter incorporating such applicator

(30) Priorité: 24.02.1994 FR 9402111
(43) Date de publication de la demande: 30.08.1995
(73) Titulaire: PORGES, 92350 Le Plessis-Robinson (FR)
(72) Inventeur: Annycke, Delphine, F-75014 Paris (FR)
(74) Mandataire: Bonnetat, Christian

(56) Documents cités:
- EP-A- 0 569 287
- EP-A- 0 580 470
- WO-A-81/03609
- FR-A- 2 693 652
- US-A- 4 416 275
- US-A- 4 589 874

## Description

La présente invention concerne un applicateur pour un cathéter externe de collecte urinaire pour individus de sexe masculin, et un dispositif de collecte urinaire comportant ledit applicateur.

On connaît des dispositifs de collecte urinaire comportant essentiellement un cathéter externe comprenant un manchon souple destiné à être appliqué de façon étanche sur le pénis d'un patient, lequel manchon est raccordé à une partie de réception du gland, sensiblement en forme de calotte sphérique, elle-même raccordée à un tube d'évacuation de l'urine, ledit manchon étant enroulé sur lui-même préalablement à son application. Une difficulté consiste en la mise en place d'un tel cathéter du fait du manque de rigidité du pénis généralement constatée chez le patient au moment de l'application du cathéter. Pour mettre en place ce dernier, il est donc nécessaire d'utiliser un applicateur.

Pour faciliter la mise en place de tels cathéters, la demanderesse a développé deux types d'applicateurs, mis en oeuvre selon le principe suivant :
- introduction du gland dans la partie de réception prévue à cet effet,
- mise en traction de la verge, et
- déroulement du manchon.

Un premier type d'applicateur (brevet FR-A-2 690 842) comporte un support annulaire, sur lequel est enroulé le manchon, ledit support présentant au moins deux mors commandables de préhension derrière le gland pour la mise en traction de la verge.

Le deuxième type d'applicateur (brevet FR-A-2 693 651) est formé par une bague dont le diamètre initial peut être réduit afin d'être serré, de même, derrière le gland pour mettre la verge en traction et, ensuite, dérouler le manchon.

Quoique donnant toute satisfaction, ces deux applicateurs nécessitent d'utiliser un cathéter de forme spécifique. En effet, afin de faciliter l'introduction du gland, l'applicateur doit présenter un diamètre supérieur à celui du "corps" du pénis. Le cathéter doit alors présenter, de façon correspondante, deux diamètres. Ainsi, le manchon possède un diamètre conforme à celui du corps du pénis, tandis que la partie de réception du gland doit être de diamètre supérieur et d'épaisseur renforcée.

Par ailleurs, un ensemble cathéter-applicateur est décrit dans le brevet US-A-4 589 874, dans lequel l'applicateur est constitué d'une bague destinée à entourer partiellement la partie de réception du gland, et sur la partie proximale de laquelle, par rapport au pénis, un manchon, destiné à être appliqué sur ce dernier, est enroulé. Dans ce cas, la bague présente un diamètre fixe, correspondant à celui du gland, ce qui signifie que le manchon, avant utilisation, est dilaté, de façon permanente, à un diamètre légèrement plus grand, compte tenu de l'épaisseur de la bague. Ainsi, lorsque le manchon est appliqué sur le corps du pénis, de plus petit diamètre, l'étanchéité entre ce dernier et le manchon ne peut absolument pas être garantie. Par ailleurs, la partie de réception du gland doit présenter, par nature, une épaisseur et une rigidité suffisantes pour l'enfiler sur le gland, supérieures à celles du manchon, ainsi qu'un diamètre correspondant.

La présente invention a pour but d'éviter ces inconvénients, et concerne un applicateur pour un cathéter externe de collecte urinaire qui, tout en permettant une saisie sûre et efficace du pénis pour dérouler le cathéter, présente une conception évitant la mise sous tension dudit cathéter et, donc, une déformation permanente éventuelle de celui-ci, avant usage.

A cet effet, l'applicateur pour cathéter externe de collecte urinaire, ledit cathéter comportant un manchon souple destiné à être appliqué de façon étanche sur le pénis d'un patient, lequel manchon est raccordé à une partie de réception du gland, de forme tubulaire, elle-même raccordée à un tube d'évacuation de l'urine, et ledit applicateur compqrtant une bague destinée à entourer au moins partiellement ladite partie de réception du gland, à l'extrémité proximale de laquelle bague, par rapport au pénis sur lequel doit être appliqué ledit manchon, ce dernier est enroulé sur lui-même préalablement à son application, est remarquable, selon l'invention, en ce que ladite bague présente spontanément une section radiale correspondant au moins sensiblement à celle du corps du pénis sur lequel doit être appliqué ledit manchon, et en ce que ladite bague comporte des moyens pour augmenter, de façon commandable, sa section radiale, pour faciliter la mise en place de la bague et, donc, de la partie de réception du gland du cathéter, sur le gland et un évidement permettant de pincer le pénis derrière le gland.

Ainsi, le manchon du cathéter, avant usage, ne subit aucune tension pouvant entraîner une déformation permanente de celui-ci. En effet, la bague présente alors une ouverture (ou section) correspondant sensiblement à la section du corps du pénis (et au diamètre nominal du manchon). L'augmentation provoquée de la section de la bague autorise l'introduction du gland dans celle-ci, mais n'est que temporaire. Par ailleurs, le manchon et la partie de réception du gland peuvent être réalisés, grâce à la conception particulière de la bague, en une pièce d'épaisseur constante. Avantageusement, ladite bague présente un corps cylindrique non fermé en une matière élastique, avec deux parties d'extrémité en regard de plus faible largeur, délimitant, chacune, un évidement pour recevoir l'autre partie, et les moyens pour commander l'augmentation de sa section radiale comprennent deux pattes disposées, respectivement, sur lesdites parties d'extrémité et s'étendant au moins sensiblement radialement en présentant entre elles, au repos, un écartement périphérique tel que, en rapprochant lesdites pattes, il se produit une déformation de la bague augmentant sa section radiale.

Bien que pouvant être rapportées sur ladite bague, lesdites pattes radiales font de préférence partie intégrante de celle-ci.

Par ailleurs, lesdites deux pattes radiales peuvent former entre elles un angle d'environ 50° en position de repos.

Pour permettre d'enrouler le manchon du cathéter sur l'extrémité proximale de la bague, ladite patte la plus proche de l'extrémité proximale de la bague présente, avantageusement, une plus faible largeur que ladite patte la plus éloignée.

Pour pouvoir pincer manuellement le gland, l'évidement est pratiqué sur le dessous du corps de ladite bague, de façon sensiblement diamétralement opposée auxdites pattes.

De plus, le corps de ladite bague peut être prolongé par un réceptacle en forme d'auge, destiné à entourer le dessous de la partie de réception du gland du cathéter et à permettre le repliage du tube sur son extrémité libre et, ainsi, la mise en tension du manchon.

Selon une autre caractéristique de l'invention, l'extrémité proximale de ladite bague est prolongée pour permettre de replier sur lui-même ledit manchon du cathéter.

Par ailleurs, des moyens coopérants sont prévus, respectivement, sur les deux parties d'extrémité du corps de ladite bague pour maintenir les pattes en position rapprochée.

Avantageusement, lesdits moyens coopérants comportent des crans sur une desdites parties d'extrémité, tandis que la patte de l'autre partie d'extrémité présente une extension parallèlement à la largeur de la bague suffisante pour coopérer avec lesdits crans.

Les figures du dessin annexé feront bien comprendre comment l'invention peut être réalisée. Sur ces figures, des références identiques désignent des éléments semblables.

La figure 1 est une vue de côté d'un exemple de réalisation d'un applicateur pour un cathéter externe de collecte urinaire selon l'invention.

La figure 2 est une vue de face de l'applicateur de la figure 1.

La figure 3 est une vue de dessus de l'applicateur de la figure 1.

Les figures 4 à 8 illustrent les différentes étapes de la mise en place d'un cathéter externe de collecte urinaire sur le pénis d'un patient, en utilisant l'applicateur des figures 1 à 3.

La figure 9 est une vue de côté d'une première variante de l'applicateur des figures 1 à 3.

Les figures 10 et 11 sont, respectivement, une vue de face et une vue de dessus, avec arrachement partiel, d'une seconde variante de l'applicateur des figures 1 à 3.

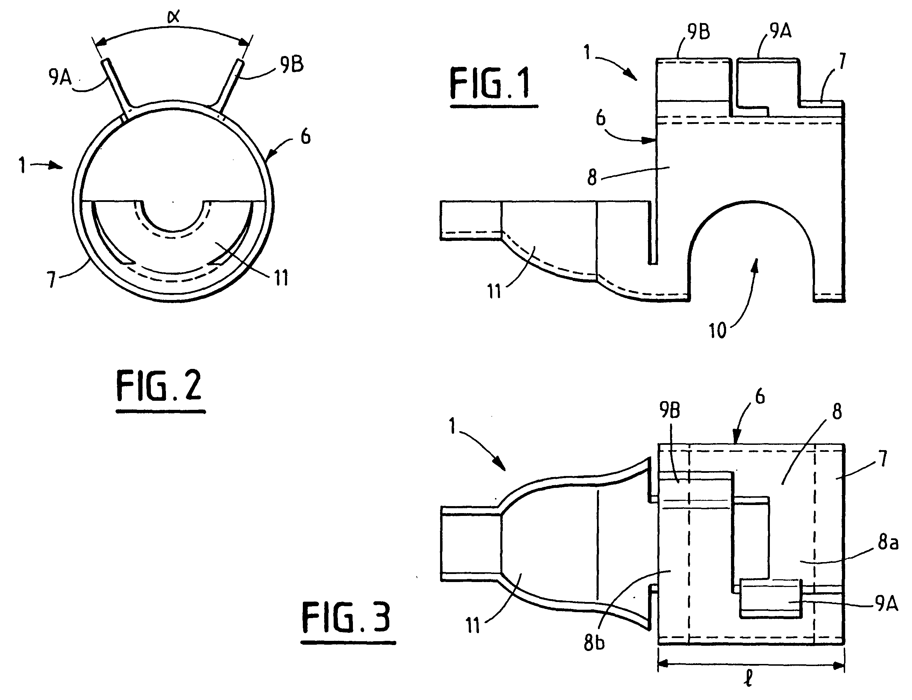

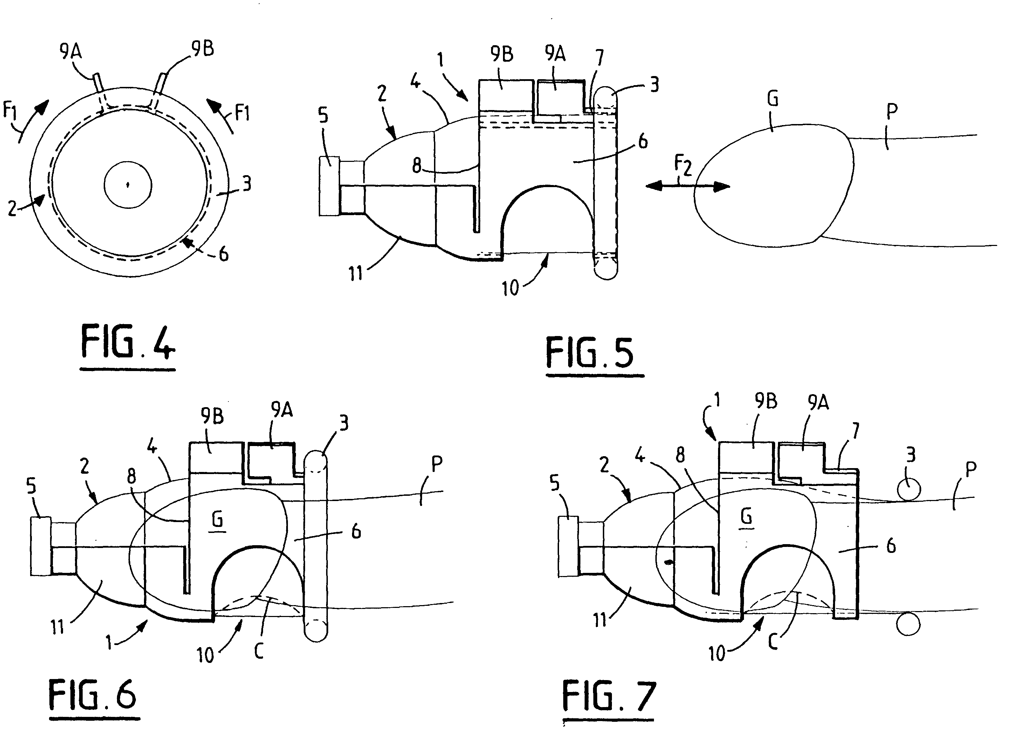

Sur les figures 1 à 3, on a représenté un exemple de réalisation d'un applicateur 1 selon l'invention pour un cathéter externe 2 de collecte urinaire, montré sur les figures 4 à 8, et 9. Ce dernier comporte, de façon classique, un manchon souple 3, destiné à être appliqué sur le pénis d'un patient P, qui est raccordé à une partie de réception 4 du gland G, de forme cylindrique, elle-même raccordée à un tube 5 d'évacuation de l'urine (pouvant être relié à des moyens de collecte de l'urine non montrés).

L'applicateur 1, quant à lui, comporte une bague 6 destinée à entourer au moins partiellement la partie de réception 4 du gland G du cathéter 2, et à l'extrémité proximale 7 de laquelle, par rapport au pénis P sur lequel doit être appliqué le manchon 3, ce dernier est enroulé sur lui-même préalablement à son application (figure 5 notamment).

Selon l'invention, la bague 6 présente spontanément une section radiale (ou transversale, figure 2) correspondant au moins sensiblement à celle du corps du pénis P (gland G exclu) sur lequel doit être appliqué le manchon 3. De plus, ladite section radiale de la bague 6 peut être augmentée de façon commandable pour faciliter la mise en place de la bague 6 et, donc, de la partie de réception 4 du gland du cathéter 2, sur le gland G.

Plus particulièrement, la bague 6 présente un corps cylindrique 8 non fermé en une matière élastique, avec deux parties d'extrémité en regard 8a,8b de plus faible largeur (correspondant, à chaque fois, à sensiblement la moitié de la largeur 1, ou extension longitudinale, de la bague), délimitant, chacune, un évidement pour la réception de l'autre partie. De plus, la bague 6, pour commander l'augmentation de sa section radiale, comprend deux pattes 9A,9B disposées, respectivement, sur les parties d'extrémité 8a,8b du corps 8 de la bague et s'étendant au moins sensiblement radialement. Les pattes 9A,9B peuvent soit être rapportées sur la bague, soit, de préférence, faire partie intégrante de celle-ci. Par ailleurs, elles présentent entre elles, au repos, un écartement périphérique tel que, en rapprochant lesdites pattes, il se produit une déformation de la bague augmentant sa section radiale. En position de repos, comme montré sur la figure 2, les pattes radiales 9A,9B forment entre elles un angle α d'environ 50°.

Comme montré sur la figure 3, la patte 9A proche de l'extrémité proximale 7 de la bague 6 présente une plus faible largeur que la patte 9B pour ainsi dégager l'emplacement destiné à recevoir le manchon enroulé 3 du cathéter.

Ainsi, l'applicateur selon l'invention se présente sous forme d'une bague de section variable (ouverture de la bague). Les deux pattes permettent, en les rapprochant, d'agrandir l'ouverture de la bague, de dilater le manchon 3 enroulé sur l'extrémité proximale 7 de celle-ci, et de faciliter, grâce à cela, l'introduction du gland dans la partie de réception 4. De ce fait, le manchon 3, en général en latex et ainsi mis en tension, ne subit pas de modification permanente, avant usage, de sa forme.

Par ailleurs, un évidement 10 est pratiqué sur le dessous du corps 8 de la bague 6, c'est-à-dire de façon sensiblement diamétralement opposée aux pattes 9A,9B. Comme on le verra plus en détail par la suite, cette découpe permet, une fois le gland introduit, de pincer le pénis derrière le gland pour le mettre en traction et, ensuite, de dérouler le manchon.

Pour maintenir le gland, après introduction dans l'applicateur, le corps 8 de la bague 6 est prolongé par un réceptacle 11 en forme d'auge, destiné à entourer le dessous de la partie de réception 4 du gland G (voir la figure 6 notamment), et à permettre le repliage du tube 5 sur son extrémité libre et, ainsi, la mise en tension du manchon 3 pour faciliter son ouverture.

Les figures 4 à 8 montrent les différentes étapes pour la mise en place du cathéter de collecte urinaire 2 sur le pénis P d'un patient à l'aide de l'applicateur 1 des figures 1 à 3.

Initialement, le manchon 3 est enroulé sur l'extrémité proximale 7 de la bague 6, enfilée par ailleurs sur la partie 4 de réception du gland du cathéter 2, dont le réceptacle 11 maintient la partie inférieure la plus proche du tube 5 d'évacuation de l'urine.

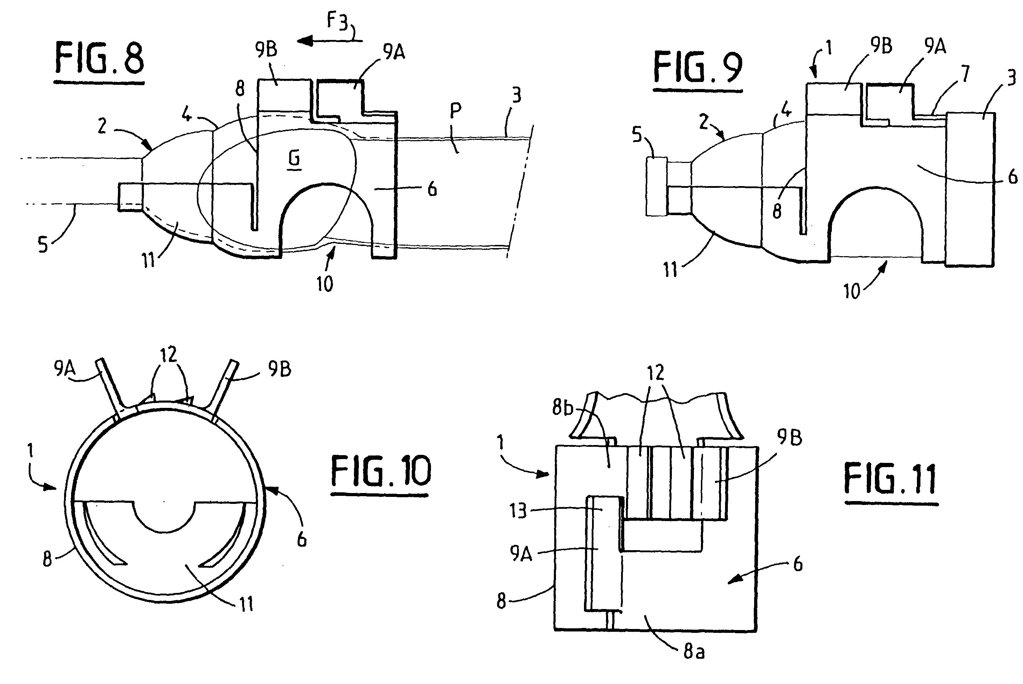

Au repos, la bague 6, dont les pattes 9A,9B sont spontanément écartées, comme montré sur la figure 1, de l'angle α, présente une section correspondant sensiblement à celle du corps du pénis P et, ainsi, le manchon 3, réalisé en une seule pièce avec la partie 4 et le tube 5, n'est pas sous tension. On évite ainsi une éventuelle déformation permanente de celui-ci. En rapprochant les pattes 9A,9B l'une de l'autre, comme montré sur la figure 4 (flèches F1), on augmente l'ouverture (section) de la bague 6 pour permettre l'introduction du gland G dans la partie de réception 4, par déplacement relatif de la bague et du pénis (figure 5, flèche F2). Une fois le gland G en place dans la partie 4 et après avoir pincé le gland G par action manuelle à travers l'évidement 10 pour permettre une traction sur le pénis, ce qui est symbolisé par la courbe C en traits interrompus sur la figure 6, on peut commencer le déroulement du manchon 3 sur le pénis P. Le manchon 3, qui n'a subi qu'une tension temporaire, revient naturellement à son diamètre d'origine pour s'appliquer, avec une étanchéité parfaite sur le corps du pénis P (figure 7). Une fois le déroulement du manchon achevé, il ne reste plus qu'à déplier le tube 5 et retirer l'applicateur 1 (figure 8, flèche F3).

Conformément à la première variante, montrée sur la figure 9, de l'applicateur 1 des figures 1 à 3, l'extrémité proximale 7 de la bague 6 est prolongée pour permettre un repli du manchon 3 vers l'extérieur et non plus son enroulement. Une bande adhésive à double face, protégée par un papier protecteur, peut être ainsi appliquée sur la partie repliée du manchon. La mise en oeuvre de l'applicateur consiste alors, après avoir appuyé sur les pattes 9A,9B pour écarter le manchon 3 et après avoir introduit le gland, à retirer le papier protecteur de la bande adhésive et à commencer le dépliage du manchon 3. Ce début de dépliage permet à la bande adhésive de coller rapidement sur le pénis, ce qui rend plus facile le dépliage ultérieur du manchon.

Conformément à la seconde variante, montrée sur les figures 10 et 11, de l'applicateur 1 des figures 1 à 3, des moyens coopérants 12,13 sont prévus, respectivement, sur les deux parties d'extrémité 8a,8b du corps 8 de la bague 6 pour maintenir les pattes 9A,9B en position rapprochée (plus grande ouverture de la bague), c'est-à-dire sans qu'il soit nécessaire pour cela d'exercer une pression manuelle sur ces dernières. Plus précisément, lesdits moyens coopérants peuvent comporter des crans 12 sur la partie d'extrémité 8b, tandis que la patte 9A de la partie d'extrémité 8a présente une extension 13 parallèlement à la largeur l de la bague 6 suffisante pour coopérer avec les crans 12, ou inversement. Cela présente deux avantages : d'une part, la main de l'opérateur peut changer de position pour pouvoir pincer le gland grâce à l'évidement prévu à cet effet et, d'autre part, un même applicateur peut être utilisé pour différentes tailles de manchon.

## Revendications

1. Applicateur pour cathéter externe de collecte urinaire, ledit cathéter comportant un manchon souple destiné à être appliqué de façon étanche sur le pénis d'un patient, ledit manchon étant enroulé sur lui-même et étant raccordé à une partie de réception du gland, de forme tubulaire, elle-même raccordée à un tube d'évacuation de l'urine, et ledit applicateur comportant une bague (6) qui est destinée à entourer au moins partiellement ladite partie de réception du gland et à l'extrémité proximale de laquelle se trouve ledit manchon (3);
**caractérisé en ce que** ladite bague (6) présente spontanément une section radiale correspondant au moins sensiblement à celle du corps du pénis sur lequel doit être appliqué ledit manchon (3), et **en ce que** ladite bague (6) comporte :
- des moyens (9A, 9B) pour augmenter, de façon commandable, sa section radiale, pour faciliter la mise en place de la bague (6) et, donc, de la partie de réception du gland (4) du cathéter (2), sur le gland ; et
- un évidement (10) permettant de pincer le pénis derrière le gland.

2. Applicateur selon la revendication 1,
**caractérisé en ce que** ladite bague (6) présente un corps cylindrique (8) non fermé en une matière élastique, avec deux parties d'extrémité en regard (8a, 8b) de plus faible largeur, délimitant, chacune, un évidement pour recevoir l'autre partie, et **en ce que** les moyens pour commander l'augmentation de sa section radiale comprennent deux pattes (9A, 9B) disposées, respectivement, sur lesdites parties d'extrémité (8a, 8b) et s'étendant au moins sensiblement radialement en présentant entre elles, au repos, un écartement périphérique tel que, en rapprochant lesdites pattes, il se produit une déformation de la bague augmentant sa section radiale.

3. Applicateur selon la revendication 2,
**caractérisé en ce que** lesdites pattes radiales (9A, 9B) font partie intégrante de ladite bague (6).

4. Applicateur selon la revendication 2 ou la revendication 3,
**caractérisé en ce que** lesdites deux pattes radiales (9A, 9B) forment entre elles un angle (α) d'environ 50° en position de repos.

5. Applicateur selon l'une quelconque des revendications 2 à 4,
**caractérisé en ce que** ladite patte (9A) la plus proche de l'extrémité proximale (7) de la bague (6) présente une plus faible largeur que ladite patte (9B) la plus éloignée.

6. Applicateur selon l'une quelconque des revendications 2 à 5,
**caractérisé en ce que** ledit évidement (10) permettant de pincer le pénis derrière le gland est pratiqué sur le dessous du corps (8) de ladite bague (6), de façon sensiblement diamétralement opposée auxdites pattes (9A, 9B).

7. Applicateur selon l'une quelconque des revendications 1 à 6,
**caractérisé en ce que** le corps (8) de ladite bague (6) est prolongé par un réceptacle (11) en forme d'auge, destiné à entourer le dessous de la partie de réception du gland (4) du cathéter (2) et à permettre le repliage du tube (5) sur son extrémité libre et, ainsi, la mise en tension du manchon (3).

8. Applicateur selon l'une quelconque des revendications 1 à 7,
**caractérisé en ce que** l'extrémité proximale (7) de ladite bague (6) est prolongée pour permettre de replier sur lui-même ledit manchon (3) du cathéter.

9. Applicateur selon l'une quelconque des revendications 2 à 8,
**caractérisé en ce que** des moyens coopérants (12, 13) sont prévus, respectivement, sur les deux parties d'extrémité (8a, 8b) du corps (8) de ladite bague (6) pour maintenir les pattes (9A, 9B) en position rapprochée.

10. Applicateur selon la revendication 9,
**caractérisé en ce que** lesdits moyens coopérants comportent des crans (12) sur une desdites parties d'extrémité (8b, 8a), tandis que la patte (9A ou 9B) de l'autre partie d'extrémité (8a, 8b) présente une extension (13) parallèlement à la largeur de la bague (6) suffisante pour coopérer avec lesdits crans (12).

11. Dispositif de collecte urinaire, comprenant un cathéter externe (2) comportant un manchon souple (3) destiné à être appliqué de façon étanche sur le pénis d'un patient, lequel manchon (3) est raccordé à une partie de réception du gland (4), de forme tubulaire, elle-même raccordée à un tube (5) d'évacuation de l'urine, ledit manchon (3) étant enroulé sur lui-même préalablement à son application,
**caractérisé en ce qu'**il comprend un applicateur (1) tel que défini dans l'une quelconque des revendications 1 à 10.

## Patentansprüche

1. Anlegevorrichtung für einen externen Harnsammelkatheter, wobei der Katheter eine elastische Manschette umfasst, die dafür vorgesehen ist, dicht am Penis eines Patienten angebracht zu werden, wobei die Manschette auf sich selbst aufgerollt und mit einem röhrenförmigen Aufnahmeteil für die Eichel verbunden ist, das wiederum mit einer Röhre zur Abführung des Harns verbunden ist, und wobei die Anlegevorrichtung einen Ring (6) umfasst, der dafür vorgesehen ist, den Aufnahmeteil für die Eichel wenigstens teilweise zu umgeben und an dessen proximalem Ende sich die Manschette (3) befindet; **dadurch gekennzeichnet, dass** der Ring (6) spontan einen radialen Querschnitt aufweist, der wenigstens im Wesentlichen demjenigen des Körpers des Penis entspricht, an welchem die Manschette (3) zu befestigen ist, und dass der Ring (6) aufweist:
- Mittel (9A, 9B), um steuerbar den radialen Querschnitt zu vergrößern, um das Anbringen des Rings (6) und somit des Aufnahmeteils (4) des Katheters (2) für die Eichel an der Eichel zu erleichtern; und
- eine Aussparung (10), die gestattet, den Penis hinter der Eichel zu fassen.

2. Anlegevorrichtung nach Anspruch 1, **dadurch gekennzeichnet, dass** der Ring (6) einen nicht geschlossenen, zylindrischen Körper (8) aus elastischem Material mit zwei einander gegenüberstehenden Endteilen (8a, 8b) mit geringerer Breite aufweist, die jeweils eine Aussparung zur Aufnahme des anderen Teils aufweisen, und dass die Mittel zur Steuerung der Vergrößerung des radialen Querschnitts zwei Laschen (9A, 9B) umfassen, die entsprechend an den Endteilen (8a, 8b) angeordnet sind und sich wenigstens im Wesentlichen radial erstrecken, wobei sie in Ruhe zwischen sich einen Abstand in Umfangsrichtung aufweisen, so dass beim Annähern der Laschen eine Verformung des Rings entsteht, die seinen radialen Querschnitt vergrößert.

3. Anlegevorrichtung nach Anspruch 2, **dadurch gekennzeichnet, dass** die radialen Laschen (9A, 9B) einen Bestandteil des Rings (6) bilden.

4. Anlegevorrichtung nach Anspruch 2 oder Anspruch 3, **dadurch gekennzeichnet, dass** die beiden radialen Laschen (9A, 9B) in der Ruheposition zwischen sich einen Winkel (α) von etwa 50° aufweisen.

5. Anlegevorrichtung nach einem der Ansprüche 2 bis 4, **dadurch gekennzeichnet, dass** die dem proximalen Ende (7) des Rings (6) nächstliegende Lasche (9A) eine geringere Breite als die weiter entferntere Lasche (9B) aufweist.

6. Anlegevorrichtung nach einem der Ansprüche 2 bis 5, **dadurch gekennzeichnet, dass** die Aussparung (10), die gestattet, den Penis hinter der Eichel zu fassen, an der Unterseite des Körpers (8) des Rings (6) im Wesentlichen den Laschen (9A, 9B) diametral entgegengesetzt angeordnet ist.

7. Anlegevorrichtung nach einem der Ansprüche 1 bis 6, **dadurch gekennzeichnet, dass** der Körper (8) des Rings (6) durch eine trogförmige Aufnahme (11) verlängert ist, die dafür vorgesehen ist, die Unterseite des Aufnahmeteils (4) des Katheters (2) für die Eichel zu umgeben und das Umschlagen der Röhre (5) an ihrem freien Ende und somit den Körperschluss der Manschette (3) zu gestatten.

8. Anlegevorrichtung nach einem der Ansprüche 1 bis 7, **dadurch gekennzeichnet, dass** das proximale Ende (7) des Rings (6) verlängert ist, um zu gestatten, die Manschette (3) des Katheters auf dasselbe umzuschlagen.

9. Anlegevorrichtung nach einem der Ansprüche 2 bis 8, **dadurch gekennzeichnet, dass** an den beiden Endteilen (8a, 8b) des Körpers (8) des Rings (6) entsprechend zusammenwirkende Mittel (12, 13) vorgesehen sind, um die Laschen (9A, 9B) in angenäherter Position zu halten.

10. Anlegevorrichtung nach Anspruch 9, **dadurch gekennzeichnet, dass** die zusammenwirkenden Mittel auf einem der Endteile (8b, 8a) Einkerbungen (12) aufweist, während die Lasche (9A oder 9B) des anderen Endteils (8a, 8b) eine Verlängerung (13) parallel zur Breite des Rings (6) aufweist, die ausreicht, um mit den Einkerbungen (12) zusammenzuwirken.

11. Harnsammelvorrichtung, die einen externen Katheter (2) umfasst, der eine elastische Manschette (3) aufweist, die dafür vorgesehen ist, dicht am Penis eines Patienten angebracht zu werden, welche Manschette (3) mit einem rohrförmigen Aufnahmeteil (4) für die Eichel verbunden ist, das wiederum mit einer Röhre (5) zur Abführung des Harns verbunden ist, wobei die Manschette (3) vor ihrem Anlegen auf sich selbst aufgerollt ist, **dadurch gekennzeichnet, dass** sie eine Anlegevorrichtung (1) wie in einem der Ansprüche 1 bis 10 definiert umfasst.

## Claims

1. Applicator for an external urinary collection catheter, said catheter comprising a flexible sheath intended to be applied in a leaktight manner onto the penis of a patient, said sheath being rolled up on itself and being connected to a glans-receiving part of tubular shape which is itself connected to a tube for removing urine, and said applicator comprising a ring (6) which is intended to at least partially enclose said glans-receiving part and at the proximal end of which said sheath (3) is situated,
**characterized in that** said ring (6) spontaneously has a radial cross section corresponding at least substantially to that of the body of the penis on which said sheath (3) is to be applied, and **in that** said ring (6) comprises:
- means (9A, 9B) for increasing its radial cross section in a controllable manner in order to make it easier to place the ring (6), and thus the glans-receiving part (4) of the catheter (2), on the glans; and
- a recess (10) allowing the penis to be gripped behind the glans.

2. Applicator according to Claim 1, **characterized in that** said ring (6) has an unclosed cylindrical body (8) of elastic material with two opposite end parts (8a, 8b) of smaller width, each delimiting a recess for receiving the other part, and **in that** the means for controlling the increase in its radial cross section comprise two tabs (9A, 9B) arranged respectively on said end parts (8a, 8b) and extending at least substantially radially and, when at rest, having between them a peripheral spacing which is such that, by moving said tabs towards each other, a deformation of the ring takes place which increases its radial cross section.

3. Applicator according to Claim 2, **characterized in that** said radial tabs (9A, 9B) form an integral part of said ring (6).

4. Applicator according to Claim 2 or Claim 3, **characterized in that** said two radial tabs (9A, 9B) between them form an angle (α) of about 50° in the rest position.

5. Applicator according to any one of Claims 2 to 4, **characterized in that** said tab (9A) nearer the proximal end (7) of the ring (6) has a smaller width than said tab (9B) farther away.

6. Applicator according to any one of Claims 2 to 5, **characterized in that** said recess (10) allowing the penis to be gripped behind the glans is formed on the bottom of the body (8) of said ring (6), in a manner substantially diametrically opposite said tabs (9A, 9B).

7. Applicator according to any one of Claims 1 to 6, **characterized in that** the body (8) of said ring (6) is continued by a trough-shaped receptacle (11) which is intended to enclose the bottom of the glans-receiving part (4) of the catheter (2) and to allow the tube (5) to be folded on its free end and thus permit tensioning of the sheath (3).

8. Applicator according to any one of Claims 1 to 7, **characterized in that** the proximal end (7) of said ring (6) is continued in order to allow said sheath (3) of the catheter to be folded on itself.

9. Applicator according to any one of Claims 2 to 8, **characterized in that** cooperating means (12, 13) are provided respectively on the two end parts (8a, 8b) of the body (8) of said ring (6) to maintain the tabs (9A, 9B) in the close position.

10. Applicator according to Claim 9, **characterized in that** said cooperating means comprise catches (12) on one of said end parts (8b, 8a), while the tab (9A or 9B) of the other end part (8a, 8b) has an extension (13) parallel to the width of the ring (6) and sufficient to cooperate with said catches (12).

11. Urinary collection device, comprising an external catheter (2) which has a flexible sheath (3) intended to be applied in a leaktight manner onto the penis of a patient, which sheath (3) is connected to a glans-receiving part (4) of tubular shape which is itself connected to a tube (5) for removal of urine, said sheath (3) being rolled up on itself prior to its application, **characterized in that** it comprises an applicator (1) as defined in any one of Claims 1 to 10.
